# EUROPEAN PATENT APPLICATION

(11) **EP 4 778 889 A1**
(43) Date of publication of application: **22.07.2026**
(21) Application number: 25876070.1
(22) Date of filing: 15.10.2025
(51) Int. Cl.: C01F 7/442, A61K 8/26, A61Q 1/02, C09C 1/40, C09D 7/60, C09D 7/61, C09D 201/00

(54) **PLATE-LIKE ALUMINA POWDER, METHOD FOR PRODUCING SAME, AND COATING MATERIAL OR COSMETIC**

(30) Priority: 22.10.2024 JP 2024185920
(71) Applicant: Nippon Light Metal Co., Ltd., Minato-ku Tokyo 105-0004 (JP)
(72) Inventor: HAYASHI, Daichi, Shizuoka-shi, Shizuoka 424-0901 (JP); YOSHIOKA, Masayoshi, Shizuoka-shi, Shizuoka 424-0901 (JP); MOCHIZUKI, Chitose, Shizuoka-shi, Shizuoka 424-0901 (JP); IDA, Takeo, Shizuoka-shi, Shizuoka 424-0901 (JP)
(74) Representative: Plasseraud IP
(86) International application number: PCT/JP2025/036266
(87) International publication number: WO 2026/088828

(57) **Abstract**

Provided are a platelet alumina powder having a lower level of spotty shine (undesirable sparkle) than conventional platelet alumina powder even when it has a particle size equal to that of the conventional one; and a method for producing such a platelet alumina powder. The platelet alumina powder includes platelet α-alumina particles having an average particle size of 2 µm or more and 100 µm or less and an average thickness of 0.2 µm or more and 3.0 µm or less as measured by SEM observation. The platelet α-alumina particles are polycrystalline. They each have a plate surface with an outer perimeter L1 and a total grain boundary length L2 and have an average L2/L1 ratio of 0.30 or more and 2.00 or less.

## Description

### TECHNICAL FIELD

The present invention relates to a platelet alumina powder and a method for producing a platelet alumina powder. The present invention also relates to a paint and a cosmetic.

### BACKGROUND ART

Alumina (Al₂O₃) is chemically stable, highly heat-, corrosion-, and wear-resistant, highly insulating, and highly strong and hard. With such advantages, alumina powder is widely used in various applications such as structural members, tools, abrasives, fillers, spark plugs, insulators, electronic substrates, and refractories.

In particular, platelet alumina powder has high particle surface light reflectance and high particle surface brightness. With such advantages, therefore, platelet alumina powder is used as a pigment for paints and cosmetics. Platelet alumina powder, which includes platelet alumina particles, is also called scaly alumina powder, flaky alumina powder, flat alumina powder, or alumina flakes.

Patent Document 1 discloses a platelet alumina powder for use as a pigment. Patent Document 1 discloses Al₂O₃ flakes having a thickness of 500 nm or more, a D₅₀ value of 15 to 30 µm, and a D₉₀ value of 30 to 45 µm and states that the Al₂O₃ flakes simultaneously have high chemical stability, smooth surfaces, and high whiteness, and are useful as a pigment base (see claim 1 and paragraphs [0010] and [0015] of Patent Document 1). Patent Document 1 also discloses that the Al₂O₃ flakes are coated with a high refractive index layer, such as TiO₂, or a low refractive index layer, such as SiO₂, which result in effective pigments having increased gloss and further increased interference color or color flop (see paragraphs [0040] to [0046] of Patent Document 1).

Patent Document 2 discloses a luminous pigment-containing paint composition containing alumina flakes as a luminous pigment. Patent Document 2 states that the composition is suitable for use as an automotive topcoat paint and capable of forming, without compromising the finished appearance, a new highly-decorative composite coating with a luminous effect stronger than ever (see claim 1 and paragraphs [0001] and [0108] of Patent Document 2). Patent Document 2 also discloses that the alumina flakes include aluminum oxide (Al₂O₃) coated with a metal oxide such as titanium dioxide and have a particle size of 10 to 30 µm and a thickness of 0.3 to 0.4 µm (see paragraph [0007] of Patent Document 2).

Patent Document 3 discloses a pearlescent pigment comprising flaky alumina crystals that are coated with metal or metal precursor particles and are composed mainly of aluminum oxide and zinc oxide in a mass ratio of 100:0.1-5 (see claim 1 of Patent Document 3). Patent Document 3 states that the crystals have an average particle thickness of 0.5 µm or less, an average particle diameter of 15 µm or more, and an aspect ratio of 50 or more and thus is superior in gloss (see paragraph [0001] of Patent Document 3).

Patent Document 4 discloses a method for producing a platelet alumina-based powder characterized by having the crystal structure of α, β, or γ-alumina alone or two or more crystal structures thereof, the method comprising: calcining platelet boehmite particles at 400°C to 1500°C (see claim 2 of Patent Document 4). Patent Document 4 also discloses that the platelet boehmite particles or platelet alumina powder may be surface-coated with a hydrophobic compound such as polysiloxane and that the coated powder may be added to cosmetics to impart a pleasant feel (see paragraph [0013] of Patent Document 4).

Patent Document 5 discloses hexagonal alumina platelets that result from calcining hexagonal boehmite platelets at a temperature of 450 to 1500°C and have substantially hexagonal plate shapes, a ratio of long axis length to short axis length of 1 to 1.3, and an aspect ratio of 40 to 100. Patent Document 5 states that the hexagonal alumina platelets are highly oriented, less diffuse, and more luminous and are also suitable for use as a filler for luminous paints or cosmetics (see claim 4 and paragraph [0045] of Patent Document 5).

Patent Document 6 discloses platelet alumina particles characterized by having a thickness of 0.01 to 5 µm, an average particle diameter of 0.1 to 500 µm, an aspect ratio (a ratio of particle diameter to thickness) of 2 to 500, and a polygonal plate shape, and containing molybdenum (see claim 1 of Patent Document 6). Patent Document 6 states that the platelet alumina particles are suitable for use as thermally conductive fillers, cosmetic materials, abrasives, highly luminous pigments, lubricants, electrically-conductive powder bases, ceramic materials, etc. (see paragraph [0101] of Patent Document 6).

### Citation List

### Patent Documents

Patent Document 1: Japanese Unexamined Patent Application, Publication No. 2014-218424
Patent Document 2: Japanese Unexamined Patent Application, Publication No. H10-298458
Patent Document 3: Japanese Unexamined Patent Application (Translation of PCT Application), Publication No. 2010-502774
Patent Document 4: Japanese Unexamined Patent Application,
Publication No. 2012-071996
Patent Document 5: Japanese Unexamined Patent Application, Publication No. 2003-002642
Patent Document 6: Japanese Unexamined Patent Application, Publication No. 2019-123664

### DISCLOSURE OF THE INVENTION

### Problems to be Solved by the Invention

Although research has been ongoing to improve the luminous effect of platelet alumina powder as described above, conventional platelet alumina powder has room for further improvement. Specifically, a platelet alumina powder with larger particle size can effectively exhibit higher reflectance.

Unfortunately, a platelet alumina powder with too large particle size may exhibit a high level of spotty shine (undesirable sparkle). As used herein, the term "spotty shine" means that the powder has localized brighter portions (brighter spots). For example, a platelet alumina powder including particles with brightness levels varying significantly with their orientation (the angle between the particles and the view direction) may have certain sites exhibiting higher brightness. Such spotty shine (undesirable sparkle) is undesirable for some applications. In particular, cosmetic applications tend to avoid pigments with spotty shine.

In view of the problem described above, the inventors have conducted intensive research. As a result, the inventors have created a platelet alumina powder having a specific average particle size, a specific average particle thickness, and a specific polycrystalline state, and have found that such a platelet alumina powder has a lower level of spotty shine (undesirable sparkle) than the conventional platelet alumina powder even when it has a particle size equal to that of the conventional one.

The present invention has been completed based on such findings. It is an object of the present invention to provide a platelet alumina powder having a lower level of spotty shine (undesirable sparkle) than the conventional platelet alumina powder even when it has a particle size equal to that of the conventional one; and to provide a method for producing such a platelet alumina powder.

### Means for Solving the Problems

The present invention encompasses aspects (1) to (8) below. As used herein, a phrase consisting of numerical values and "to" therebetween means the range between the numerical values (inclusive). In other words, the phrase "X to Y" is interchangeable with "X or more and Y or less".

(1) A platelet alumina powder including a plurality of platelet α-alumina particles having an average particle size of 2 µm or more and 100 µm or less and an average thickness of 0.2 µm or more and 3.0 µm or less as measured by scanning electron microscope (SEM) observation,
   the platelet α-alumina particles being polycrystalline,
   the platelet α-alumina particles each having a plate surface with an outer perimeter L1 and a total grain boundary length L2,
   the platelet α-alumina particles having an average L2/L1 ratio of 0.30 or more and 2.00 or less.
(2) The platelet alumina powder according to aspect (1), wherein the platelet α-alumina particles have an average L2/L1 ratio of 0.50 or more and 1.50 or less.
(3) The platelet alumina powder according to aspect (1) or (2), wherein the platelet α-alumina particles have an average particle size of 5 µm or more and 50 µm or less and an average thickness of 0.3 µm or more and 1.0 µm or less.
(4) A method for producing the platelet alumina powder according to any one of aspects (1) to (3), the method including the steps of:
   preparing a raw material mixture including an aluminum hydroxide powder and an additive; and
   calcining the raw material mixture by holding the raw material mixture at a temperature in the range of 1000°C or more and 1300°C or less for a time period of less than 20 hours,
   wherein the raw material mixture contains 0.01 mass% or more and 0.5 mass% or less of an alkali metal (AM) on an AM₂O basis, 0.1 mass% or more and 0.3 mass% or less of silicon (Si) on an SiO₂ basis, and 0.1 mass% or more and 5.0 mass% or less of fluorine (F) on an F basis, and
   wherein the step of calcining the raw material mixture includes increasing temperature at a rate of 50°C/hour or more and 150°C/hour or less.
(5) The method according to aspect (4), wherein the additive includes an alkali silicofluoride (AM₂SiF₆), or aluminum fluoride (AlF₃) and silicon oxide (SiO₂).
(6) The method according to aspect (5), wherein the additive further includes one or both of an alkali metal (AM) oxide (AM₂O) and an alkali metal (AM) carbonate (AM₂CO₃).
(7) The method according to any one of aspects (4) to (6), wherein the alkali metal (AM) is one or both of sodium (Na) and potassium (K).
(8) A paint or cosmetic including the platelet alumina powder according to any one of aspects (1) to (3).

### Effects of the Invention

The present invention provides a platelet alumina powder having a lower level of spotty shine (undesirable sparkle) than conventional platelet alumina powder even when it has a particle size equal to that of the conventional one, and provides a method for producing such a platelet alumina powder.

### BRIEF DESCRIPTION OF THE DRAWINGS

FIG. 1 shows how a platelet alumina powder produces a luminous effect;
FIG. 2 schematically shows how light is scattered through a polycrystalline material; and
FIG. 3 schematically shows the plate surface of a platelet particle.

### PREFERRED MODE FOR CARRYING OUT THE INVENTION

Hereinafter, specific embodiments of the present invention (hereinafter referred to as "embodiments") will be described. It will be understood that the embodiments described below are not intended to limit the present invention and may be altered or modified in various ways without departing from the gist of the present invention. It will also be understood that the specification will cover any combination of preferred modes technically consistent with one another. For example, the specification will cover any combination of two or more preferred numerical ranges.

### 1. Platelet Alumina Powder

The platelet alumina powder according to an embodiment (hereinafter also simply referred to as "the alumina powder") includes a plurality of platelet α-alumina (A1₂O₃) particles (hereinafter also simply referred to as "alumina particles", "platelet particles", or "particles"). Specifically, the platelet alumina powder includes platelet α-alumina particles as a main component. As used herein, the term "platelet α-alumina particles" refers to particles including α-alumina (corundum). α-Alumina, which has a trigonal corundum crystal structure, is chemically stable, highly heat-, corrosion-, and wear-resistant, highly insulating, and highly strong and hard. α-Alumina also exhibits high light reflectance, high brightness, and high whiteness. The alumina powder according to an embodiment, which includes platelet α-alumina particles as a main component, can fully take advantage of such features.

As used herein, the term "powder" means a collection of many independent particles. In other words, a powder includes a collection of many particles. A powder including only particles independent from one another can have fluidity as a whole. A powder should include particles mostly independent from one another. A powder may contain an aggregate of some particles as long as it has fluidity as a whole. A collection of many particles dispersed in a medium, such as a liquid or resin, may also be called "powder".

The platelet alumina powder according to an embodiment includes alumina particles each having a plate shape (scaly, lamellar, flat, or flaky shape). Specifically, each of the particles has a relatively large plate surface and a relatively small thickness. Specifically, the platelet alumina powder has an average particle size of 2 µm or more and 100 µm or less and an average particle thickness of 0.2 µm or more and 3.0 µm or less. As used herein, the term "average particle size" refers to the number average of the sizes of particles in the alumina powder, and the term "average thickness" or "average particle thickness" refers to the number average of the thicknesses of particles in the alumina powder. The term "particle size" refers to the long axis length of a particle. Specifically, the term "particle size" refers to the long axis length of the plate surface of a particle. The term "thickness" refers to the thickness of a platelet. The particle size and thickness can be determined by scanning electron microscope (SEM) observation of particles in the alumina powder. In other words, the average particle size and the average thickness are the values determined by SEM observation. In general, the average particle size is larger than the average thickness.

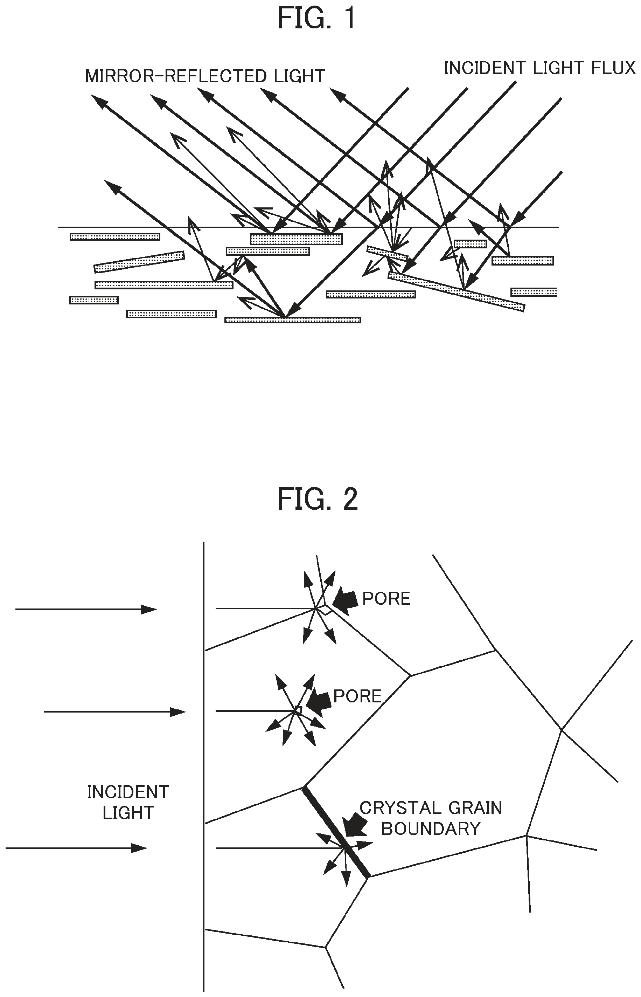

The alumina powder including particles having such a large plate surface and such a small thickness can have a high luminous effect. This feature will be described with reference to FIG. 1. As shown in FIG. 1, when a powder including particles with large plate surfaces and small particle thicknesses is applied onto a base material by coating or any other method, larger plate surfaces of the particles will tend to be oriented parallel to the base material surface. In addition, larger size particles have larger plate surface areas oriented parallel to the base material surface. Light from outside is reflected by the plate surfaces of the particles to outside. Thus, larger size particles can reflect more incident light on their plate surfaces to exhibit a higher luminous effect to the viewer.

With an average particle size of less than 2 µm or an average particle thickness of more than 3.0 µm, the powder may exhibit an insufficient luminous effect. With an average particle size of more than 100 µm or an average particle thickness of less than 0.2 µm, the powder may be difficult to produce. The powder with such an average particle size or such an average particle thickness may be low in strength or difficult to handle. The platelet alumina powder preferably has an average particle size of 5 µm or more and 50 µm or less and an average particle thickness of 0.3 µm or more and 1.0 µm or less.

The alumina powder preferably has an average aspect ratio of 20 or more and 50 or less. In this regard, the average aspect ratio is the ratio of the average particle size to the average particle thickness (average thickness) (average particle size/average particle thickness (average thickness)). With an average aspect ratio of 20 or more, the alumina powder can exhibit a higher luminous effect. With an average aspect ratio of 50 or less, the alumina powder can have higher strength and be easier to handle.

It should be noted that not all the particles in the alumina powder have to be platelet α-alumina particles. The alumina powder may contain particles other than platelet α-alumina particles as long as it satisfies the requirements for average particle size and average particle thickness as a whole. However, the alumina powder preferably has a higher platelet α-alumina particle content so that it can take advantage of the excellent performance of platelet α-alumina particles. The alumina powder preferably has a platelet α-alumina particle content of 80 mass% or more, more preferably 90 mass% or more, even more preferably 95 mass% or more.

In the alumina powder according to an embodiment, the platelet α-alumina particles are polycrystalline. Specifically, each of the particles is not one single crystal but includes a plurality of crystallites (or crystal grains). In each of the particles, adjacent crystallites are in contact with each other with a grain boundary between them. In addition, the platelet α-alumina particles each have a plate surface with an outer perimeter L1 and a total grain boundary length L2, and the platelet α-alumina particles have an average L2/L1 ratio of 0.30 or more and 2.00 or less. The alumina powder including such polycrystalline alumina particles with a ratio (L2/L1) of total grain boundary length to outer perimeter in such a specific range can have a reduced level of spotty shine (undesirable sparkle).

This feature will be described with reference to FIG. 2, which schematically shows the scattering of light passing through a polycrystalline material. In an alumina particle having a polycrystalline structure, each of the crystallites in the polycrystalline structure has a uniform crystal structure and exhibits a uniform refractive index. Thus, light travels straight through each crystallite without being scattered. However, the crystal structure is disordered at the grain boundary and may even be amorphous. Thus, the grain boundary causes the refractive index to vary. Thus, at the grain boundary, light does not travel straight and can be scattered in all directions. Consequently, this reduces the reflected light intensity and undesirable sparkle. In contrast, single-crystalline alumina particles can reflect more light with less scattering and thus exhibit highly undesirable sparkle.

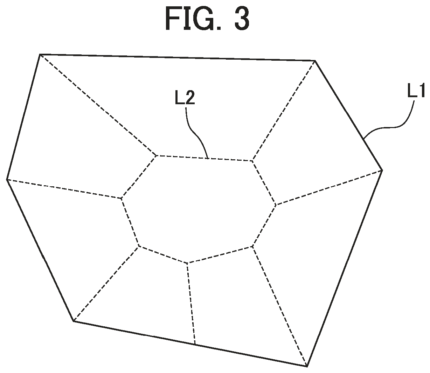

Each of the particles with a controlled content of grain boundary can have a controlled level of spotty shine (undesirable sparkle). The grain boundary content can be estimated from the ratio (L2/L1) of the total grain boundary length (L2) to the outer perimeter (L1) of the particle. In this regard, as shown in FIG. 3, the outer perimeter (L1) of the particle is the length of the outer circumference of the plate surface of the platelet particle. The total grain boundary length (L2) is the sum of the lengths of grain boundaries exposed on the plate surface of the platelet particle. In this regard, FIG. 3 schematically shows the plate surface of the platelet particle. In FIG. 3, L1 is shown as a solid line, and L2 is shown as a broken line. L2/L1 is the ratio of the total grain boundary length (L2) to the outer perimeter (L1) of each particle. The average L2/L1 ratio is the average of the L2/L1 ratios of two or more particles.

With an average L2/L1 ratio of 0.30 or more and 2.00 or less, the particles can have an appropriate grain boundary content and thus exhibit increased luminous effect and reduced undesirable sparkle. In contrast, particles with an average L2/L1 ratio of less than 0.30 may have an insufficient grain boundary content and thus be not effective in exhibiting a reduced level of light-scattering-induced undesirable sparkle. Particles with an average L2/L1 ratio of more than 2.00 may have too high a grain boundary content and thus produce more scattered light and less reflected light, which may cause the problem of loss of luminous effect. To exhibit both high luminous effect and reduced undesirable sparkle, the particles preferably have an average L2/L1 ratio of 0.40 or more and 1.80 or less, more preferably 0.50 or more and 1.50 or less.

Whether the platelet α-alumina particles are polycrystalline or not can be determined by SEM observation of the particles. The average L2/L1 ratio can also be determined by SEM observation of the platelet alumina powder. Specifically, when the surface or cross-section of the particles is observed by SEM, crystal grain boundaries are observed, which makes it possible to confirm that the platelet α-alumina particles are polycrystalline. The L2/L1 ratio, determined as the ratio of crystal grain boundary length to outer perimeter on the particle surface, may be used to evaluate the degree of polycrystallinity.

When measured in an oriented state, the platelet alumina powder according to an embodiment should preferably have a powder X-ray diffraction (XRD) pattern with an α-alumina diffraction peak intensity ratio in a specific range. Specifically, the platelet alumina powder preferably has an I_{A}/I_{B} ratio of 0.3 or more and 10 or less, in which I_{A} is the (006) plane diffraction peak intensity of α-alumina, and I_{B} is the (113) plane diffraction peak intensity of α-alumina. The platelet alumina powder preferably has an I_{C}/I_{B} ratio of 4.0 or more and 10 or less, in which I_{C} is the (104) plane diffraction peak intensity of α-alumina, and I_{B} is the (113) plane diffraction peak intensity of α-alumina. Moreover, the platelet alumina powder preferably has an I_{D}/I_{B} ratio of 3.0 or more and 7.5 or less, in which I_{D} is the (116) plane diffraction peak intensity of α-alumina, and I_{B} is the (113) plane diffraction peak intensity of α-alumina. The platelet alumina powder also preferably has an I_{E}/I_{B} ratio of 1.0 or more and 7.5 or less, in which I_{E} is the (018) plane diffraction peak intensity of α-alumina, and I_{B} is the (113) plane diffraction peak intensity of α-alumina. The platelet alumina powder also preferably has an I_{F}/I_{B} ratio of 5.0 or more and 20.0 or less, in which I_{F} is the (1010) plane diffraction peak intensity of α-alumina, and I_{B} is the (113) plane diffraction peak intensity of α-alumina.

The (006) plane of α-alumina corresponds to the crystal plane (c-plane) perpendicular to the c-axis of the α-alumina crystal. The (104), (116), (018), and (1010) planes of α-alumina are all crystal planes making a small angle with the c plane (c plane-like crystal planes). The platelet alumina powder according to an embodiment includes platelet α-alumina particles each having a large plate surface and a small thickness. Therefore, when filled in a measurement holder for the measurement of powder XRD pattern, the platelet α-alumina particles will be oriented to have their plate surfaces aligned with the measuring plane. Thus, the XRD pattern of the measured platelet alumina powder will have high diffraction peak intensities (I_{A}, I_{C}, I_{D}, I_{E}, and I_{F}) for the c plane (plate surface) and (006), (104), (116), (018), and/or (1010) plane (c plane-like crystal plane).

The XRD pattern of the platelet alumina powder in the oriented state can be determined as described below. The powder sample is placed on a glass sample plate and pressed so as to form a flat sample surface before being measured with an XRD analyzer. When placed on the sample plate, the platelet particles in the powder sample can be easily oriented to have a specific crystal axis aligned with the sample plate surface (the occurrence of selective orientation effect). If the platelet alumina powder is ground before the preparation of the measurement sample, the particles will have changed shapes, which may impair the selective orientation effect. Thus, the sample should not be modified in particle size before the measurement. As used herein, the term "peak intensity" means a peak area (integrated intensity). The XRD pattern measured using a CuKα X-ray source will have a (006) plane diffraction peak of α-alumina at 2θ = 41.7 ± 0.5°. The XRD pattern will also have a (113) plane diffraction peak at 2θ = 43.3 ± 0.5°. The XRD pattern will also have a (104) plane diffraction peak at 2θ = 35.1 ± 0.5°. The XRD pattern will also have a (116) plane diffraction peak at 2θ = 57.5 ± 0.5°. The XRD pattern will also have a (018) plane diffraction peak at 2θ = 61.3 ± 0.5°. The XRD pattern will also have a (1010) plane diffraction peak at 2θ = 76.9 ± 0.5°.

The alumina powder according to an embodiment preferably contains 0.01 mass% or more and 0.5 mass% or less of an alkali metal (AM) on an AM₂O basis and 0.01 mass% or more and 0.3 mass% or less of silicon (Si) on an SiO₂ basis. The alumina powder more preferably has an alkali metal (AM) content of 0.01 mass% or more and 0.3 mass% or less on an AM₂O basis. The alumina powder more preferably has a silicon (Si) content of 0.01 mass% or more and 0.2 mass% or less on an SiO₂ basis. The alumina powder preferably contains one or both of sodium (Na) and potassium (K) as the alkali metal (AM). The alumina powder containing such components can provide a higher luminous effect. The alumina powder more preferably contains 0.01 mass% or more and 0.5 mass% or less of sodium (Na) on an Na₂O basis. The alumina powder with such features can exhibit a more effectively reduced level of undesirable sparkle.

The alumina powder according to an embodiment preferably includes at least aluminum (Al), alkali metal (AM), silicon (Si), and oxygen (0). However, it may contain other components (elements other than Al, AM, Si, and O) as long as it satisfies the requirements described above. It may also contain impurities unavoidably introduced during the manufacturing process. Such other components include fluorine (F). Fluorine (F) may be a component derived from an additive introduced during the production of the alumina powder. Specifically, as described later, an additive containing fluorine (F) may be added during the production of the alumina powder. The added fluorine may partially remain while it is almost vaporized during the calcining step. The alumina powder typically has a fluorine content of 0.5 mass% or less, 0.3 mass% or less, or 0.1 mass% or less.

The alumina powder having a high content of other components (elements other than Al, AM, Si, and O) may have a reduced level of luminous effect. Thus, the alumina powder preferably has a content of other components of 10 mass% or less, more preferably 5 mass% or less, even more preferably 1 mass% or less, furthermore preferably 0.5 mass% or less, most preferably 0.1 mass% or less. In particular, iron (Fe) can act to color the alumina powder. Thus, the alumina powder preferably contains as small an amount of iron (Fe) as possible. The alumina powder preferably has an iron (Fe) content of 0.05 mass% or less on an iron oxide (Fe₂O₃) basis.

The alumina powder according to an embodiment preferably has a specific surface area (S_{BET}) of 0.1 m²/g or more and 5.0 m²/g or less, more preferably 0.3 m²/g or more and 2.0 m²/g or less. With too large a specific surface area, the alumina powder may have a small particle size and thus may exhibit a reduced level of luminous effect. With a small specific surface area, the alumina powder may be difficult to produce.

The alumina powder according to an embodiment, which includes α-alumina as a main component, has high chemical stability. The alumina powder according to an embodiment has a large average particle size and high dispersibility. The alumina powder with such features is suitable for use in the fields of paints and cosmetics. However, the alumina powder according to an embodiment can find applications other than paints and cosmetics. The alumina powder according to an embodiment is also suitable for use as a plastic- or resin film-reinforcing material, a plastic- or resin film-forming gas barrier material, a structural member, a tool material, an abrasive, a filler, a spark plug material, an insulator, an electronic substrate material, a refractory, or any other suitable material in known applications.

### 2. Method for Producing Platelet Alumina Powder

The platelet alumina powder according to an embodiment may be produced using any suitable method capable of producing a product satisfying the requirements described above. However, the platelet alumina powder according to an embodiment should preferably be produced using the procedure described below. A preferred method for producing the platelet alumina powder includes the steps of: preparing a raw material mixture including an aluminum hydroxide powder and an additive (raw material mixing step); and calcining the raw material mixture by holding the raw material mixture at a temperature in the range of 1000°C or more and 1300°C or less for a time period of less than 20 hours (calcining step). The raw material mixture contains 0.01 mass% or more and 0.5 mass% or less of an alkali metal (AM) on an AM₂O basis, 0.1 mass% or more and 0.3 mass% or less of silicon (Si) on an SiO₂ basis, and 0.1 mass% or more and 5.0 mass% or less of fluorine (F) on an F basis. The step of calcining the raw material mixture includes increasing temperature at a rate of 50°C/hour or more and 150°C/hour or less. If necessary, the calcining step may be followed by a post-processing step(s).

Such a production method according to an embodiment is characterized by using an aluminum hydroxide powder as a raw material, controlling the composition of the raw material mixture within a specific range, and controlling the calcining conditions within a specific range. Such characteristics allow the production of an alumina powder that has a desired particle size and a polycrystalline state and thus has a high luminous effect and a reduced level of spotty shine (undesirable sparkle). Hereinafter, each of the steps will be described in detail.

### Raw Material Mixing Step

The raw material mixing step includes preparing a raw material mixture including an aluminum hydroxide powder and an additive. Aluminum hydroxide is a compound represented by the chemical formula Al(OH)₃. When heated, it dehydrates and transforms into α-aluminum oxide (α-alumina). Aluminum hydroxide is known to have two forms: gibbsite (γ-aluminum hydroxide) and bayerite (α-aluminum hydroxide). In an embodiment, one or both of gibbsite and bayerite may be used. However, the aluminum hydroxide powder preferably includes gibbsite, which is more thermodynamically stable.

The aluminum hydroxide powder (raw material) may have any particle size that allows the production of the alumina powder according to an embodiment. However, the aluminum hydroxide powder preferably has a volume average particle size (D50) of 0.5 µm or more and 15 µm or less. Alternatively, the aluminum hydroxide powder should have a specific surface area (S_{BET}) of 0.5 m²/g or more and 20 m²/g or less. In this regard, D50 is the cumulative 50% diameter of the volume particle size distribution determined using a laser diffraction/scattering particle size distribution analyzer. S_{BET} is the value measured by N₂ gas adsorption method.

The additive may be a component that will form a liquid phase to serve as a mineralizer in the subsequent calcining step. The additive includes at least silicon (Si) and fluorine (F) and optionally includes alkali metal (AM). As described later, appropriate control of the proportion of the liquid phase components allows alumina particle crystallization and grain growth to proceed appropriately, which will result in the production of an alumina powder having a desired particle size and a polycrystalline state.

Thus, appropriate control of the composition of the raw material mixture containing the additive is important for the production of the desired alumina powder. Specifically, the proportion of the additive in the raw material mixture may be controlled such that the resulting raw material mixture will contain 0.01 mass% or more and 0.5 mass% or less of an alkali metal (AM) on an AM₂O basis, 0.1 mass% or more and 0.3 mass% or less of silicon (Si) on an SiO₂ basis, and 0.1 mass% or more and 5.0 mass% or less of fluorine (F) on an F basis. The use of the raw material mixture having the composition within such a range enables the production of an alumina powder that has a desired particle size and a polycrystalline state and thus has high luminous effect and reduced undesirable sparkle. On the other hand, the raw material mixture with an inappropriate composition may have an adverse effect on the particle size and the crystalline state.

The raw material mixture more preferably has an alkali metal (AM) content of 0.01 mass% or more and 0.5 mass% or less on an AM₂O basis. The raw material mixture preferably contains one or both of sodium (Na) and potassium (K) as the alkali metal, and more preferably contains sodium (Na). The raw material mixture more preferably has a silicon (Si) content of 0.10 mass% or more and 0.20 mass% or less on an SiO₂ basis. The raw material mixture more preferably has a fluorine (F) content of 0.1 mass% or more and 1.0 mass% or less on an F basis. The raw material mixture with a composition limited within the specified range can form an alumina powder having a more favorable polycrystalline state.

The additive includes at least silicon (Si) and fluorine (F). The additive may or may not contain alkali metal (AM). The aluminum hydroxide powder (raw material) may contain alkali metal (AM) as an impurity. If the aluminum hydroxide powder has a sufficient alkali metal content, the additive does not need to contain alkali metal. On the other hand, if the aluminum hydroxide powder has an insufficient alkali metal content, the additive should preferably contain alkali metal. The additive may also contain aluminum (Al). Aluminum in the additive will be incorporated into the alumina powder during the calcining step.

The additive may contain any additional component other than alkali metal (AM), silicon (Si), fluorine (F), aluminum (Al), and oxygen (O) as long as the raw material mixture can form an alumina powder having a desired particle size and a polycrystalline state. However, too high a content of such an additional component may have an adverse effect on the particle size and the polycrystalline state. The additive preferably has an additional component content of 20 mass% or less, more preferably 10 mass% or less, even more preferably 5 mass% or less, furthermore preferably 1 mass% or less. The additive does not need to contain such an additional component (other than alkali metal (AM), silicon (Si), fluorine (F), aluminum (Al), and oxygen (O)) in an amount exceeding the impurity level.

In a preferred mode, the additive includes aluminum fluoride (AlF₃) and silicon oxide (SiO₂). In a case where the aluminum hydroxide powder has an insufficient alkali metal content, the additive should preferably include one or both of an alkali metal (AM) oxide (AM₂O) and an alkali metal (AM) carbonate (AM₂CO₃). In another preferred mode, the additive includes an alkali silicofluoride (AM₂SiF₆). The use of an appropriate amount of the additive including such compounds (AlF₃, SiO₂, AM₂O, AM₂CO₃, AM₂SiF₆) enables reliable production of an alumina powder having a desired particle size and a polycrystalline state. Moreover, these compounds are inexpensive and readily available. Thus, the use of these compounds allows lower-cost production of the platelet alumina powder.

The raw material mixture may be prepared by mixing the aluminum hydroxide powder and the additive. The materials may be mixed using any known method. The materials may be mixed by a dry or wet mixing method. The dry mixing method may be performed using a dry mixer, such as an air blender, a V-type blender, a rocking blender, a Henschel mixer, or a Nauta mixer. The wet mixing method may include mixing the aluminum hydroxide powder and the additive with a solvent such as water to form a slurry; and mixing the resulting slurry using a wet mixer, such as a ball mill, an attritor, or a bead mill.

### Calcining Step

The calcining step includes calcining the resulting raw material mixture by holding the resulting raw material mixture at a temperature in the range of 1000°C or more and 1300°C or less for a time period of less than 20 hours. The calcining step produces a calcined product. During the calcination, aluminum hydroxide (Al(OH)₃) dehydrates and transforms into α-alumina (Al₂O₃). During the calcination, the alkali metal (AM), silicon (Si), and fluorine (F) in the raw material mixture form an oxidized liquid phase, which serves as a mineralizer to promote the crystallization of α-alumina. Thus, the crystal structure changes during the transformation of Al(OH)₃ to α-alumina. The mineralizer added promotes the crystallization of α-alumina (corundum), which allows the transformation to proceed at a lower temperature.

The components (AM, Si, F) of the mineralizer, which form the liquid phase during the calcination, can cover the surfaces of the alumina particles and partially enter the particles. The components of the liquid phase can serve to accelerate particle diffusion and growth. Specifically, some of the aluminum (Al) atoms can dissolve from the alumina particle surface into the liquid phase and then form precipitates on other sites of the particle surface. This leads to the growth of alumina particles. During this process, the alumina particle growth direction and growth rate will differ depending on the proportion of the liquid phase components. Thus, appropriate control of the proportion of the liquid phase components will lead to appropriate control of the growth of alumina particle plate surface, which will result in the production of highly flat particles having a desired polycrystalline state. The components of the liquid phase are also effective in forming smooth plate surfaces for the alumina particles. Any irregularity portions on alumina particle surfaces can preferentially diffuse and disappear into the liquid phase. Thus, appropriate control of the proportion of the liquid phase components allows the production of alumina particles having smooth surfaces, desired sizes, and polycrystalline state.

If calcined at a temperature of less than 1000°C, the raw material cannot sufficiently transform into α-alumina and may undergo insufficient particle growth. That may make it difficult to produce an alumina powder having high dispersibility. If calcined at a temperature of more than 1300°C, the particles may be sintered together to form a solid sintered mass. In such a case, the calcination may also consume too much energy, which may lead to increased production costs. The raw material mixture is preferably calcined at a temperature of 1000°C or more and 1200°C or less, more preferably 1100°C or more and 1200°C or less. The raw material mixture is preferably calcined for a time period of 10 hours or more and less than 20 hours. Under such conditions, the particles can be prevented from undergoing excessive calcination and can reliably undergo transformation into α-alumina and particle growth. The raw material mixture may be calcined using any calcining furnace that allows the production of the desired alumina powder. In order to allow the additive to function effectively during the calcination, however, the furnace is preferably a stationary furnace having a closed-type calcining vessel.

In the production method according to an embodiment, the calcining step includes increasing temperature at a rate of 50°C/hour or more and 150°C/hour or less. In addition to the proportion of the liquid phase components and the calcining temperature, the rate of temperature increase during the calcination is important for the control of the polycrystalline state of the alumina powder. The larger (higher) the rate of temperature increase, the greater the grain boundary length will be. Thus, the higher the rate of temperature increase, the greater the ratio (L2/L1) of the total grain boundary length (L2) to the outer perimeter (L1) of the alumina particle will be. On the other hand, the smaller (lower) the rate of temperature increase, the smaller the ratio (L2/L1) will be. The control of the proportion of the liquid phase components within the specified range, the control of the calcining temperature within the specified range, and the control of the rate of temperature increase within the specified range enable the control of the ratio (L2/L1) within the desired range and thus enable the production of an alumina powder having a reduced level of spotty shine (undesirable sparkle). It should be noted that the rate of temperature increase described above is used when the temperature is in the range of at least 25°C (room temperature) or more and 1000°C or less.

### Post-Processing Step

If necessary, the calcined product resulting from the calcining step may be subjected to a post-processing step(s), such as an alkali removal step, a disintegration step, and/or a classification step. The alkali removal step may include removing an excess of alkali metal components from the surface of the calcined product. This step can control the alkali metal content of the final alumina powder product. For example, the alkali metal components on the particle surface can be removed by washing the calcined product with water and filtering the washed product. The disintegration step may include applying a small amount of mechanical energy to the calcined product to break the bonding between aggregated particles resulting from the calcination. The disintegration step may be performed by a dry or wet method using a disintegrator, such as a pot mill, a pin mill, and/or a jaw crusher. The classification step may include classifying the particles according to size to yield an alumina powder with a desired particle size. The classification step may be performed using sieving, air classification, water elutriation, and/or centrifugal separation. The post-processing step may be performed as necessary. When the desired alumina powder results from the calcining step, the post-processing step may be omitted.

The process described above successfully produces the platelet alumina powder according to an embodiment. The resulting alumina powder has smooth particle surfaces, desired particle sizes, and polycrystalline states. Thus, the resulting alumina powder has a high luminous effect and a reduced level of spotty shine (undesirable sparkle). The alumina powder with such features is suitable for use in various applications, including paints and cosmetics.

### 3. Paint and Cosmetic

An embodiment is directed to a paint or cosmetic including the platelet alumina powder described above. The paint or cosmetic may contain the alumina powder in its pure form or in a surface-treated form. The surface treatment may include treating the particle surfaces of the platelet alumina powder with a surface treatment agent, such as a silicone compound, an alkylsilane compound, and/or a fluorine-containing compound. The paint or cosmetic may contain a solvent or a resin in addition to the platelet alumina powder. The solvent may be aqueous or non-aqueous. The paint or cosmetic may further contain a known additive, such as an oil agent, a pigment other than the platelet alumina powder, a filler, a surfactant, a viscosity adjuster, a preservative, a fragrance, a moisturizer, a bioactive component, a salt, a chelating agent, a neutralizing agent, and/or a pH adjuster.

### EXAMPLES

The present invention will be further described in detail with reference to examples and comparative examples below. It will be understood that the examples are not intended to limit the present invention.

### (1) Preparation of Platelet Alumina Powders

### Examples 1 to 5 and Comparative Example 1

An aluminum hydroxide (gibbsite) powder (Nippon Light Metal Co., Ltd.) was provided as an alumina source for Examples 1 to 3 and Comparative Example 1. Another aluminum hydroxide (gibbsite) powder (Nippon Light Metal Co., Ltd.) was provided as an alumina source for Examples 4 and 5. The aluminum hydroxide powders have the properties shown in Table 1 below. In Table 1, the "Na₂O" column indicates the total of the content of soluble sodium at and near the aluminum hydroxide crystal surface and the content of insoluble sodium incorporated in the crystal lattice, and the "f-Na₂O" column indicates the content of only soluble sodium at and near the aluminum hydroxide crystal surface.

Separately, the following additives were provided: aluminum fluoride (AlF₃, DO-FLUORIDE CHEMICALS CO., LTD.), silicon oxide (SiO₂, Snowmark Silica Stone SP-3, Marukama Kamado, Inc.), sodium carbonate (Na₂CO₃, Kanto Chemical Co., Inc.), and sodium silicofluoride (sodium hexafluorosilicate (Na₂SiF₆), Kanto Chemical Co., Inc.).

The aluminum hydroxide powder and the additives were mixed to form a raw material mixture. The amounts of the additives were adjusted such that the raw material mixture had each additive content shown in Table 1 below. The materials were mixed manually (a manual method including putting the materials into a bag; and shaking the bag (Examples 1, 2, 4, and 5)) or by a method using a rocking mixer (a mechanical method (Example 3 and Comparative Example 1)).

### Calcining Step

The resulting raw material mixture was loaded into a calcining vessel, which was then closed with a dedicated lid. The lidded calcining vessel was placed inside a calcining furnace and subjected to calcination in air. The raw material mixture was calcined using an electric furnace or a shuttle kiln. During the calcination, the raw material mixture was heated at a rate of temperature increase of 100°C/hour to the calcining temperature and then held at the calcining temperature for 10 hours (Examples 1 to 5) or 20 hours (Comparative Example 1), which was followed by natural cooling. The calcining temperature was 1100°C (Examples 1 to 5) or 1050°C (Comparative Example 1). After the furnace temperature dropped completely, the calcining vessel was removed from the furnace, and the calcined product (alumina) was recovered.

### Post-Processing Steps

The resulting calcined product was subjected to post-processing steps. The calcined product was subjected to wet disintegration with a strength adjusted not to destroy the plate shape and then subjected to filtration to give a disintegrated product (alumina). The disintegrated product was then dried at 105°C overnight using a drying machine. After the disintegration step, coarse and fine particles were removed from the product.

As described above, the platelet alumina powders of Examples 1 to 5 and Comparative Example 1 were obtained. Table 1 below summarizes the conditions for production of the platelet alumina powders.

### Comparative Example 2

In Comparative Example 2, a platelet alumina powder (YFA10030, Kinsei Matec Co., Ltd.,) was obtained from the commercial source and evaluated for properties.

**[Table 1]**

| Table 1: Conditions for production of platelet alumina powder | | | | | | | | |
|---|---|---|---|---|---|---|---|---|
| | | Example | | | | | Comparative Example | |
| | | 1 | 2 | 3 | 4 | 5 | 1 | 2 |
| Alumina source | Type | Aluminum hydroxide (gibbsite) | | | | | | Alumina |
| | Na₂O (mass%) | 0.3 | 0.2 | 0.3 | 0.01 | 0.01 | 0.2 | - |
| | f-Na₂O (mass%) | 0.01 | 0.01 | 0.01 | <0.01 | <0.01 | 0.01 | - |
| | SiO₂ (mass%) | 0.01 | 0.01 | 0.01 | <0.01 | <0.01 | 0.01 | - |
| | Fe₂O₃ (mass%) | 0.01 | 0.01 | 0.01 | 0.01 | 0.01 | 0.01 | - |
| | S_{BET} (m²/g) | 5.0 | 4.9 | 4.7 | 1.5 | 1.5 | 4.8 | - |
| | D50 (µm) | 1.2 | 1.2 | 1.3 | 3.4 | 3.4 | 1.2 | - |
| Additive content | AlF₃ (mass%) | - | 0.6 | 0.6 | 1.2 | 1.2 | 0.6 | - |
| | SiO₂ (mass%) | - | 0.15 | 0.10 | 0.20 | 0.10 | 0.05 | - |
| | Na₂CO₃ (mass%) | - | - | - | - | - | - | - |
| | Na₂SiF₆ (mass%) | 0.7 | - | - | - | - | - | - |
| Raw material mixing | | ^{*1} Manual | | ^{*2} Mechanical | Manual | | Mechanical | - |
| Calcining conditions | Rate of temperature (°C/h) increase | 100 | | | | | 100 | - |
| | Temperature (°C) | 1100 | | | | | 1050 | - |
| | Holding time (h) | 10 | | | | | 20 | - |
| | Furnace type | Electric furnace | Shuttle kiln | | | Electric furnace | Shuttle kiln | - |
| | Vessel | Sagger | | | | | | - |
| Post-processing step | | Disintegration Coarse particle removal Fine particle removal | Disintegration Coarse particle removal Fine particle removal | Disintegration Coarse particle removal Fine particle removal | Disintegration Coarse particle removal Fine particle removal | Disintegration Coarse particle removal Fine particle removal | Disintegration Coarse particle removal Fine particle removal | - |

| | | | | | | | | |
|---|---|---|---|---|---|---|---|---|
| *1: "Manual" means a manual mixing method including putting the raw materials into a bag and shaking the bag. *2: "Mechanical" means a method including mixing the raw materials using a rocking mixer. *3: "-" means that the measurement or step was not performed. | | | | | | | | |

### (2) Evaluation

The platele alumina power samples obtained in Examples 1 to 5 and Comparative Examples 1 and 2 were each subjected to the evaluation of various properties as described below.

### Particle Size Distribution

The alumina powder was measured for particle size distribution using a laser diffraction/scattering particle size distribution analyzer (Microtrac MT3300, Nikkiso Co., Ltd.). Specifically, the alumina powder was directly introduced into the inlet port of the analyzer. After being dispersed for one minute using the dispersing function equipped in the analyzer, the alumina powder was measured for particle size distribution. The average particle size (D50) was determined as the cumulative 50 vol% particle size of the particle size distribution.

### SEM Observation

The alumina powder was evaluated using a scanning electron microscope (SEM JSM-F100, JEOL Ltd.) and image analysis software (ImageJ). Specifically, the SEM image of the alumina powder was captured, in which the particle images (platelet α-alumina particle images) were evaluated using ImageJ for the determination of particle sizes and particle thicknesses. The average particle size and the average particle thickness were calculated from the measured values. The average particle size was the average of the measurements of 80 particles, and the average particle thickness was the average of the measurements of 30 particles. The average aspect ratio was calculated as the ratio of the average particle size to the average particle thickness (average particle size/average particle thickness).

Electron backscatter diffraction (EBSD) analysis was performed to analyze the crystalline state of the particles (platelet α-alumina particles) in the alumina powder. Specifically, the EBSD analysis was performed using a scanning electron microscope (SEM JSM-6490A, JEOL Ltd.) equipped with an electron microscope crystal orientation analyzer and an analysis tool (OIM EBSD System, TSL Solutions) to observe the crystal morphology of alumina. During the analysis, the plate surface of a particle was observed, and the outer perimeter (L1) of the plate surface, the total grain boundary length (L2) on the plate surface, and the long axis length (L3) of the particle were measured, from which L2/L1 and L2/L3 were calculated. The same procedure was performed on 30 particles, and the measurements were averaged to yield the average L2/L1 ratio and the average L2/L3 ratio.

### Crystal Phase

An X-ray diffractometer (RINT Ultima III, Rigaku Corporation) was used to analyze the crystal phase of the alumina powder. Specifically, the alumina powder (sample) was placed on a glass sample plate and pressed so as to form a flat sample surface (selective orientation). The alumina powder was then analyzed using an XRD analyzer. The X-ray source was CuKα. The resulting X-ray diffraction pattern was examined to determine whether α-alumina (corundum phase) diffraction peaks appeared or not.

For the samples showing α-alumina diffraction peaks, the intensities of the (006), (113), (104), (116), (018), and (1010) plane diffraction peaks of α-alumina were determined (represented by I_{A}, I_{B}, I_{C}, I_{D}, I_{E}, and I_{F}, respectively), from which the peak intensity ratios I_{A}/I_{B}, I_{C}/I_{B}, I_{D}/I_{B}, I_{E}/I_{B}, and I_{F}/I_{B} were calculated.

### Component Analysis

Component analysis was performed using a scanning X-ray fluorescence analyzer (ZSX Primus IV, Rigaku Corporation). Specifically, the alumina powder was placed in a platinum crucible, which was then placed in a high-frequency melting apparatus. After being preheated at 700°C for 60 seconds, the alumina powder was shaken at 1200°C for 120 seconds to form a glass bead sample. The glass bead sample was then evaluated using the scanning X-ray analyzer. The evaluation result was obtained by comparing the resulting data with the standard sample data for calibration curve.

### Specific Surface Area

The alumina powder was evaluated for specific surface area (S_{BET}) by N₂ gas adsorption method using a manual specific surface area meter (FlowSorb III 2305, Micromeritics Instrument Corp.).

### Undesirable Sparkle (Spotty Shine)

The platelet alumina powder was evaluated for undesirable sparkle. Specifically, five healthy adult testers (three males and two females) applied a suitable amount (about 1 g) of the platelet alumina powder to the back of their hand and then visually observed the applied powder. They conducted scoring based on the evaluation criteria below.

3: Spotty shine (undesirable sparkle) is at a low level.
2: Spotty shine is at a slightly high level.
1: Spotty shine is at a high level.

For each sample, the average (rounded to one decimal place) of the scores from the five testers were calculated and used to evaluate the spotty shine (undesirable sparkle).

### (3) Evaluation Results

Table 2 below summarizes the evaluation results obtained for Examples 1 to 5 and Comparative Examples 1 and 2.

The alumina powder of each of Examples 1 to 5 has an average particle size within the specified range (2 to 100 µm) according to an embodiment and has an average particle thickness within the specified range (0.2 to 3.0 µm) according to an embodiment. The alumina powder of each of Examples 1 to 5 also includes polycrystalline particles having an average ratio (L2/L1) of total grain boundary length (L2) to outer perimeter (L1) within the specified range (0.30 to 2.0) according to an embodiment. Moreover, the alumina powders of Examples of 1 to 3 have a relatively high score of 2.4 to 3.0 as a result of evaluation of spotty shine.

In contrast, the alumina powder of Comparative Example 1 has a low average L2/L1 ratio not falling within the specified range (0.30 to 2.0) according to an embodiment. Thus, the alumina powder of Comparative Example 1 has a low score of 1.2 as a result of evaluation of spotty shine. Furthermore, the alumina powder of Comparative Example 2 includes single-crystalline particles.

**[Table 2] Table 2: Properties of platelet alumina powder**

| | | Example | | | | | Comparative Example | |
|---|---|---|---|---|---|---|---|---|
| | | 1 | 2 | 3 | 4 | 5 | 1 | 2 |
| D50^{*4} | (µm) | 30 | 39 | 34 | 15 | 4.3 | 32 | 9.4 |
| Average particle size^{*5} | (µm) | 35 | 45 | 40 | 17 | 4.1 | 39 | 8.4 |
| Average particle thickness^{*5} | (µm) | 0.9 | 0.9 | 0.9 | 1.1 | 0.4 | 0.9 | 0.3 |
| Average aspect ratio^{*5} | | 39 | 52 | 46 | 15 | 9 | 42 | 27 |
| Crystal morphology | | Polycrystalline | Polycrystalline | Polycrystalline | Polycrystalline | Polycrystalline | Polycrystalline | Single-crystalline |
| L2/L1 (Average) | | 0.93 | 0.55 | 0.50 | 1.02 | 0.71 | 0.21 | 0.39 |
| L2/L3(Average) | | 2.58 | 1.65 | 1.40 | 2.79 | 1. 97 | 0.57 | 1.15 |
| **α-Alumina** detection^{*6} | | Present | | | | | | |
| I_{A}/I_{B} | | 4.2 | 4.5 | 4.3 | 0.2 | <0.1 | 4.3 | 3.2 |
| I_{C}/I_{B} | | 6.7 | 7.3 | 6.8 | 2.7 | 1.3 | 7.0 | 0.2 |
| I_{D}/I_{B} | | 3.7 | 4.0 | 4.1 | 2.3 | 1.3 | 4.8 | 2.0 |
| I_{E}/I_{B} | | 4.5 | 4.9 | 4.8 | 0.7 | 0.1 | 5.4 | 0.6 |
| I_{F}/I_{B} | | 14.9 | 13.8 | 14.5 | 2.0 | 0.3 | 17.8 | 1.7 |
| Na₂O | (mass%) | 0.1 | 0.1 | 0.1 | 0.1 | 0.1 | 0.1 | 0.2 |
| 5102 | (mass%) | 0.1 | 0.1 | 0.1 | 0.1 | 0.1 | 0.1 | 0.1 |
| Fe₂O₃ | (mass%) | 0.01 | 0.01 | 0.01 | 0.02 | 0.02 | 0.01 | 0.02 |
| S_{BET} | (m²/g) | 0.5 | 0.3 | 0.5 | 0.5 | 1.2 | 0.3 | 2.7 |
| Spotty shine | | 3.0 | 2.6 | 2.4 | - | - | 1.2 | - |

| | | | | | | | | |
|---|---|---|---|---|---|---|---|---|
| *4: The value measured using a laser diffraction/scattering particle size distribution analyzer *5: The value measured by SEM observation *6: Presence or absence of detected α-alumina (corundum phase) peak *7: "-" means that the measurement or evaluation was not performed. | | | | | | | | |

These results show that the embodiments of the present invention provide platelet alumina powders having a lower level of spotty shine (undesirable sparkle) than conventional platelet alumina powder even when they have a particle size equal to that of the conventional one, and provide methods for producing such platelet alumina powders.

## Claims

1. A platelet alumina powder comprising a plurality of platelet α-alumina particles having an average particle size of 2 µm or more and 100 µm or less and an average thickness of 0.2 µm or more and 3.0 µm or less as measured by scanning electron microscope (SEM) observation,
the platelet α-alumina particles being polycrystalline,
the platelet α-alumina particles each having a plate surface with an outer perimeter L1 and a total grain boundary length L2,
the platelet α-alumina particles having an average L2/L1 ratio of 0.30 or more and 2.00 or less.

2. The platelet alumina powder according to claim 1, wherein the platelet α-alumina particles have an average L2/L1 ratio of 0.50 or more and 1.50 or less.

3. The platelet alumina powder according to claim 1 or 2,
wherein the platelet α-alumina particles have an average particle size of 5 µm or more and 50 µm or less and an average thickness of 0.3 µm or more and 1.0 µm or less.

4. A method for producing the platelet alumina powder according to claim 1 or 2, the method comprising the steps of:
preparing a raw material mixture comprising an aluminum hydroxide powder and an additive; and
calcining the raw material mixture by holding the raw material mixture at a temperature in a range of 1000°C or more and 1300°C or less for a time period of less than 20 hours,
wherein the raw material mixture contains 0.01 mass% or more and 0.5 mass% or less of an alkali metal (AM) on an AM₂O basis, 0.1 mass% or more and 0.3 mass% or less of silicon (Si) on an SiO₂ basis, and 0.1 mass% or more and 5.0 mass% or less of fluorine (F) on an F basis, and
wherein the step of calcining the raw material mixture comprises increasing temperature at a rate of 50°C/hour or more and 150°C/hour or less.

5. The method according to claim 4, wherein the additive comprises an alkali silicofluoride (AM₂SiF₆), or aluminum fluoride (AlF₃) and silicon oxide (SiO₂).

6. The method according to claim 5, wherein the additive further comprises one or both of an alkali metal (AM) oxide (AM₂O) and an alkali metal (AM) carbonate (AM₂CO₃).

7. The method according to claim 4, wherein the alkali metal (AM) is one or both of sodium (Na) and potassium (K).

8. A paint or cosmetic comprising the platelet alumina powder according to claim 1 or 2.
